# EUROPÄISCHE PATENTSCHRIFT

(11) **EP 3 184 646 B1**
(45) Veröffentlichungstag und Bekanntmachung des Hinweises auf die Patenterteilung: **17.04.2019**
(21) Anmeldenummer: 15201815.6
(22) Anmeldetag: 22.12.2015
(51) Int. Cl.: C12Q 1/6883

(54) **SPEZIFISCHE SIGNATUREN IN ALZHEIMER DURCH MULTIZENTRISCHE MIRNA-PROFILE**
SPECIFIC SIGNATURES IN ALZHEIMER'S DISEASE VIA MULTICENTRE MIRNA PROFILES
SIGNATURES SPECIFIQUES DANS ALZHEIMER PAR PROFIL MIRNA MULTICENTIQUE

(43) Veröffentlichungstag der Anmeldung: 28.06.2017
(73) Patentinhaber: Siemens Aktiengesellschaft, 80333 München (DE)
(72) Erfinder: Keller, Andreas, 66346 Püttlingen (DE); Stähler, Cord Friedrich, 69493 Hirschberg an der Bergstraße (DE); Sickert, Daniel, 90403 Nürnberg (DE); Backes, Christina, 66125 Saarbrücken (DE)

(56) Entgegenhaltungen:
- EP-A1- 2 733 220
- WO-A1-2009/009457
- WO-A1-2013/118066
- WO-A2-2011/057003
- WO-A2-2013/003350
- HUI DONG ET AL: "Serum MicroRNA Profiles Serve as Novel Biomarkers for the Diagnosis of Alzheimer's Disease", DISEASE MARKERS., Bd. 14, Nr. 1, 20. Mai 2015 (2015-05-20), Seiten 27-11, XP055274672, GB ISSN: 0278-0240, DOI: 10.1159/000343452
- HIROSHA GEEKIYANAGE ET AL: "Blood serum miRNA: Non-invasive biomarkers for Alzheimer's disease", EXPERIMENTAL NEUROLOGY, Bd. 235, Nr. 2, 1. Juni 2012 (2012-06-01), Seiten 491-496, XP055058220, ISSN: 0014-4886, DOI: 10.1016/j.expneurol.2011.11.026
- WEBER MICHEL J: "New human and mouse microRNA genes found by homology search", FEBS JOURNAL, WILEY-BLACKWELL PUBLISHING LTD, GB, vol. 272, no. 1, 1 January 2005 (2005-01-01), pages 59-73, XP002555647, ISSN: 1742-464X, DOI: 10.1111/J.1432-1033.2004.04389.X [retrieved on 2004-12-02]

### SEQUENCE LISTING

<110> Siemens Healthcare GmbH
<120> Spezifische Signaturen in Alzheimer durch multizentrische miRNA-Profile
<130> 201519137
<160> 84
<170> PatentIn version 3.5
<210> 1
   <211> 22
   <212> RNA
   <213> Homo sapiens
<400> 1
   gcugacuccu aguccagggc uc 22
<210> 2
   <211> 21
   <212> RNA
   <213> Homo sapiens
<400> 2
   uuucccuuuc cauccuggca g 21
<210> 3
   <211> 23
   <212> RNA
   <213> Homo sapiens
<400> 3
   cuacccucgg ucugcuuacc aca 23
<210> 4
   <211> 19
   <212> RNA
   <213> Homo sapiens
<400> 4
   ucucucugac uccauggca 19
<210> 5
   <211> 22
   <212> RNA
   <213> Homo sapiens
<400> 5
   uuaucagaau cuccaggggu ac 22
<210> 6
   <211> 22
   <212> RNA
   <213> Homo sapiens
<400> 6
   cugcccuagu cuagcugaag cu 22
<210> 7
   <211> 22
   <212> RNA
   <213> Homo sapiens
<400> 7
   uuucuauuuc ucaguggggc uc 22
<210> 8
   <211> 22
   <212> RNA
   <213> Homo sapiens
<400> 8
   cacuagauug ugagcuccug ga 22
<210> 9
   <211> 21
   <212> RNA
   <213> Homo sapiens
<400> 9
   cuagacugaa gcuccuugag g 21
<210> 10
   <211> 21
   <212> RNA
   <213> Homo sapiens
<400> 10
   cuccguuugc cuguuucgcu g 21
<210> 11
   <211> 22
   <212> RNA
   <213> Homo sapiens
<400> 11
   caugccuuga guguaggacc gu 22
<210> 12
   <211> 22
   <212> RNA
   <213> Homo sapiens
<400> 12
   acugcaguga aggcacuugu ag 22
<210> 13
   <211> 22
   <212> RNA
   <213> Homo sapiens
<400> 13
   cuuucagucg gauguuugca gc 22
<210> 14
   <211> 98
   <212> RNA
   <213> Homo sapiens
<400> 14
   acccaaaccc uaggucugcu gacuccuagu ccagggcucg ugauggcugg ugggcccuga 60
   acgagggguc uggaggccug gguuugaaua ucgacagc 98
<210> 15
   <211> 110
   <212> RNA
   <213> Homo sapiens
<400> 15
   aaccauuagg gggcuguggu uugccagggc aggaggugga agggagcccc auuuacagug 60
   guaacuuccu uucccuuucc auccuggcag gcuucagaga acuuuaccag 110
<210> 16
   <211> 101
   <212> RNA
   <213> Homo sapiens
<400> 16
   gcuggggcug ggugggugug gcaggcccac cuuggguaug caaagcucug acaguguuuc 60
   acuugcuacc cucggucugc uuaccacacu cccaguucug c 101
<210> 17
   <211> 66
   <212> RNA
   <213> Homo sapiens
<400> 17
   aggauucugc cauaggaagc uuggagugga acugaccugc ccccuuucuc ucugacucca 60
   uggcag 66
<210> 18
   <211> 72
   <212> RNA
   <213> Homo sapiens
<400> 18
   ggagcuuauc agaaucucca gggguacuuu auaauuucaa aaaguccccc aggugugauu 60
   cugauuugcu uc 72
<210> 19
   <211> 85
   <212> RNA
   <213> Homo sapiens
<400> 19
   gggaagggcu ucagccaggc uagugcaguc ugcuuugugc caacacuggg gugaugacug 60
   cccuagucua gcugaagcuu uuccc 85
<210> 20
   <211> 70
   <212> RNA
   <213> Homo sapiens
<400> 20
   agugagcaac ccagugggcu auggaaaugu guggaagaug gcauuucuau uucucagugg 60
   ggcucuuacc 70
<210> 21
   <211> 86
   <212> RNA
   <213> Homo sapiens
<400> 21
   gguccuugcc cucaaggagc ucacagucua uugaguuacc uuucugacuu ucccacuaga 60
   uugugagcuc cuggagggca ggcacu 86
<210> 22
   <211> 90
   <212> RNA
   <213> Homo sapiens
<400> 22
   uuuccugccc ucgaggagcu cacagucuag uaugucucau ccccuacuag acugaagcuc 60
   cuugaggaca gggaugguca uacucaccuc 90
<210> 23
   <211> 86
   <212> RNA
   <213> Homo sapiens
<400> 23
   gguggguggu uucuccguuu gccuguuucg cugaugugca uucaacucau ucucagcaaa 60
   auaagcaaau ggaaaauucg uccauc 86
<210> 24
   <211> 91
   <212> RNA
   <213> Homo sapiens
<400> 24
   cgacuugcuu ucucuccucc augccuugag uguaggaccg uuggcaucuu aauuacccuc 60
   ccacacccaa ggcuugcaga agagcgagcc u 91
<210> 25
   <211> 84
   <212> RNA
   <213> Homo sapiens
<400> 25
   gucagaauaa ugucaaagug cuuacagugc agguagugau augugcaucu acugcaguga 60
   aggcacuugu agcauuaugg ugac 84
<210> 26
   <211> 71
   <212> RNA
   <213> Homo sapiens
<400> 26
   gcgacuguaa acauccucga cuggaagcug ugaagccaca gaugggcuuu cagucggaug 60
   uuugcagcug c 71
<210> 27
   <211> 22
   <212> RNA
   <213> Homo sapiens
<400> 27
   cuuucagucg gauguuuaca gc 22
<210> 28
   <211> 22
   <212> RNA
   <213> Homo sapiens
<400> 28
   aauguuggaa uccucgcuag ag 22
<210> 29
   <211> 17
   <212> RNA
   <213> Homo sapiens
<400> 29
   gcggggcugg gcgcgcg 17
<210> 30
   <211> 20
   <212> RNA
   <213> Homo sapiens
<400> 30
   gugcauugcu guugcauugc 20
<210> 31
   <211> 22
   <212> RNA
   <213> Homo sapiens
<400> 31
   ccaauauuac ugugcugcuu ua 22
<210> 32
   <211> 23
   <212> RNA
   <213> Homo sapiens
<400> 32
   ugagugugug ugugugagug ugu 23
<210> 33
   <211> 21
   <212> RNA
   <213> Homo sapiens
<400> 33
   ucagcuacua ccucuauuag g 21
<210> 34
   <211> 22
   <212> RNA
   <213> Homo sapiens
<400> 34
   aauugcacgg uauccaucug ua 22
<210> 35
   <211> 23
   <212> RNA
   <213> Homo sapiens
<400> 35
   ccggucccag gagaaccugc aga 23
<210> 36
   <211> 22
   <212> RNA
   <213> Homo sapiens
<400> 36
   ugagguagua gguugugugg uu 22
<210> 37
   <211> 20
   <212> RNA
   <213> Homo sapiens
<400> 37
   acuggacuug gagucagaaa 20
<210> 38
   <211> 20
   <212> RNA
   <213> Homo sapiens
<400> 38
   acugggcuug gagucagaag 20
<210> 39
   <211> 23
   <212> RNA
   <213> Homo sapiens
<400> 39
   agcuacauug ucugcugggu uuc 23
<210> 40
   <211> 20
   <212> RNA
   <213> Homo sapiens
<400> 40
   acuggacuug gagccagaag 20
<210> 41
   <211> 22
   <212> RNA
   <213> Homo sapiens
<400> 41
   uccgucucag uuacuuuaua gc 22
<210> 42
   <211> 22
   <212> RNA
   <213> Homo sapiens
<400> 42
   ugagguagua gguuguaugg uu 22
<210> 43
   <211> 23
   <212> RNA
   <213> Homo sapiens
<400> 43
   ccuccguguu accuguccuc uag 23
<210> 44
   <211> 22
   <212> RNA
   <213> Homo sapiens
<400> 44
   uuauaaagca augagacuga uu 22
<210> 45
   <211> 22
   <212> RNA
   <213> Homo sapiens
<400> 45
   uguccucuag ggccugcagu cu 22
<210> 46
   <211> 22
   <212> RNA
   <213> Homo sapiens
<400> 46
   cuauacgacc ugcugccuuu cu 22
<210> 47
   <211> 21
   <212> RNA
   <213> Homo sapiens
<400> 47
   ucacagugaa ccggucucuu u 21
<210> 48
   <211> 22
   <212> RNA
   <213> Homo sapiens
<400> 48
   ucucugggcc ugugucuuag gc 22
<210> 49
   <211> 22
   <212> RNA
   <213> Homo sapiens
<400> 49
   aaaaacugag acuacuuuug ca 22
<210> 50
   <211> 20
   <212> RNA
   <213> Homo sapiens
<400> 50
   aaaaguaauu gugguuuuug 20
<210> 51
   <211> 22
   <212> RNA
   <213> Homo sapiens
<400> 51
   aaaaguaauu gugguuuuug cc 22
<210> 52
   <211> 20
   <212> RNA
   <213> Homo sapiens
<400> 52
   aaaaguaauu gugguuuuug 20
<210> 53
   <211> 21
   <212> RNA
   <213> Homo sapiens
<400> 53
   aaaaguaauu gugguuuuug c 21
<210> 54
   <211> 23
   <212> RNA
   <213> Homo sapiens
<400> 54
   agcagcauug uacagggcua uca 23
<210> 55
   <211> 21
   <212> RNA
   <213> Homo sapiens
<400> 55
   uaaagugcug acagugcaga u 21
<210> 56
   <211> 22
   <212> RNA
   <213> Homo sapiens
<400> 56
   caaaagugau ugugguuuuu gc 22
<210> 57
   <211> 23
   <212> RNA
   <213> Homo sapiens
<400> 57
   agcagcauug uacagggcua uga 23
<210> 58
   <211> 22
   <212> RNA
   <213> Homo sapiens
<400> 58
   agagguagua gguugcauag uu 22
<210> 59
   <211> 22
   <212> RNA
   <213> Homo sapiens
<400> 59
   ugauauguuu gauauauuag gu 22
<210> 60
   <211> 21
   <212> RNA
   <213> Homo sapiens
<400> 60
   guuccugcug aacugagcca g 21
<210> 61
   <211> 22
   <212> RNA
   <213> Homo sapiens
<400> 61
   ugucuuacuc ccucaggcac au 22
<210> 62
   <211> 23
   <212> RNA
   <213> Homo sapiens
<400> 62
   aaaagugcuu acagugcagg uag 23
<210> 63
   <211> 22
   <212> RNA
   <213> Homo sapiens
<400> 63
   uacgucaucg uugucaucgu ca 22
<210> 64
   <211> 22
   <212> RNA
   <213> Homo sapiens
<400> 64
   ugugagguug gcauuguugu cu 22
<210> 65
   <211> 22
   <212> RNA
   <213> Homo sapiens
<400> 65
   uuuugugucu cccauucccc ag 22
<210> 66
   <211> 22
   <212> RNA
   <213> Homo sapiens
<400> 66
   ugagguagua guuugugcug uu 22
<210> 67
   <211> 23
   <212> RNA
   <213> Homo sapiens
<400> 67
   caaagugcuu acagugcagg uag 23
<210> 68
   <211> 22
   <212> RNA
   <213> Homo sapiens
<400> 68
   uacccauugc auaucggagu ug 22
<210> 69
   <211> 22
   <212> RNA
   <213> Homo sapiens
<400> 69
   ucuucaccug ccucugccug ca 22
<210> 70
   <211> 21
   <212> RNA
   <213> Homo sapiens
<400> 70
   uacaguacug ugauaacuga a 21
<210> 71
   <211> 22
   <212> RNA
   <213> Homo sapiens
<400> 71
   ugagguagga gguuguauag uu 22
<210> 72
   <211> 22
   <212> RNA
   <213> Homo sapiens
<400> 72
   uuggcugguc ucugcuccgc ag 22
<210> 73
   <211> 22
   <212> RNA
   <213> Homo sapiens
<400> 73
   cuggcccucu cugcccuucc gu 22
<210> 74
   <211> 21
   <212> RNA
   <213> Homo sapiens
<400> 74
   gaucucacuu uguugcccag g 21
<210> 75
   <211> 22
   <212> RNA
   <213> Homo sapiens
<400> 75
   ugagguagua gauuguauag uu 22
<210> 76
   <211> 22
   <212> RNA
   <213> Homo sapiens
<400> 76
   ugagguagua gguuguauag uu 22
<210> 77
   <211> 23
   <212> RNA
   <213> Homo sapiens
<400> 77
   cagugcaaua guauugucaa agc 23
<210> 78
   <211> 22
   <212> RNA
   <213> Homo sapiens
<400> 78
   uccccuucug caggccugcu gg 22
<210> 79
   <211> 23
   <212> RNA
   <213> Homo sapiens
<400> 79
   uaaagugcuu auagugcagg uag 23
<210> 80
   <211> 22
   <212> RNA
   <213> Homo sapiens
<400> 80
   uuccugggcu ucuccucugu ag 22
<210> 81
   <211> 21
   <212> RNA
   <213> Homo sapiens
<400> 81
   ucucucggcu ccucgcggcu c 21
<210> 82
   <211> 22
   <212> RNA
   <213> Homo sapiens
<400> 82
   ugagguagua aguuguauug uu 22
<210> 83
   <211> 22
   <212> RNA
   <213> Homo sapiens
<400> 83
   uucugccucu guccaggucc uu 22
<210> 84
   <211> 22
   <212> RNA
   <213> Homo sapiens
<400> 84
   ugagguagua guuuguacag uu 22

## Beschreibung

Die Erfindung betrifft ein Verfahren zur Diagnose von Alzheimer (AD, Alzheimer's Disease) in einem Patienten, bei dem das Expressionsprofil von miRNAs in einer Blutprobe bestimmt und ein Vergleich der Expressionsniveaus definierter miRNAs zu einer Referenzprobe durchgeführt wird, und die Verwendung dieser definierten miRNAs als Marker für die Diagnose von Alzheimer.

Alzheimer wird gewöhnlich klinisch aus der Patientenhistorie, der mittelbaren Anamnese der Verwandtschaft und anhand klinischer Beobachtungen, basierend auf der Anwesenheit charakteristischer neurologischer und neuropsychologischer Merkmale und der Abwesenheit alternativer Zustände wie anderen Erkrankungen oder vorübergehenden Zuständen wie Alkoholisierung (Ausschlussverfahren), diagnostiziert. Zudem kann moderne medizinische Bildgebung mit Computertomografie (CT), Magnetresonanztomografie (MRI, magnetic resonance imaging), mit Einzelphotonen-Emissionscomputertomographie (SPECT, single photon emission computed tomography) und/oder Positronen-Emissions-Tomografie (PET, positron emission tomography) verwendet werden, um andere zerebrale Symptome oder Unterarten von Demenz auszuschließen. Darüber hinaus kann sie den Übergang von prodromalen Phasen (leichte kognitive Beeinträchtigung, MCI (mild cognitive impairment)) zu Alzheimer vorhersagen. Wenn sie als diagnostisches Werkzeug verfügbar sind werden SPECT- und PET-Neuroimaging verwendet, um eine Alzheimerdiagnose in Verbindung mit Evaluierungen, welche eine Untersuchung des Geisteszustands beinhalten, zu bestätigen. Bei einer Person, welche bereits Demenz hat, scheint SPECT besser bei der Differenzierung zwischen Alzheimer und anderen möglichen Ursachen zu sein im Vergleich zu den gewöhnlichen Ansätzen, welche psychologische Tests und eine Analyse der Anamnese anwenden.

Fortschritte haben zu Vorschlägen neuer diagnostischer Kriterien geführt. Eine neue, als PiB PET bekannte Technik wurde entwickelt, um direkt und klar Beta-Amyloid-Ablagerungen in vivo unter Verwendung eines Tracers, der selektiv an die A-beta Ablagerungen bindet, abbilden zu können. Die PiB-PET Verbindung verwendet Kohlenstoff-11 PET-Scannen. Neuere Studien deuten an, dass PiB-PET 86% akkurat in der Vorhersage ist, welche Patienten mit einer leichten kognitiven Beeinträchtigung Alzheimer innerhalb von zwei Jahren entwickeln, und dieses Verfahren zu ca. 92% akkurat beim Ausschluss der Wahrscheinlichkeit der Entwicklung von Alzheimer ist. Es wurde angedeutet, dass die Amyloid-Tomografie zukünftig wahrscheinlich in Verbindung mit anderen Marken benutzt werden wird, und nicht als Alternative. Jüngste Studien haben gezeigt, dass Patienten mit AD verminderte Glutamat- Werte (Glu) wie auch herabgesetzte Verhältnisse von Glu/Kreatin (Cr), Glu/Myo-Inosit (mI), Glu/N-Acetylaspartat (NAA) und NAA/Cr im Vergleich zu Menschen ohne AD aufweisen. Sowohl ein vermindertes Verhältnis NAA/Cr wie auch vermindertes Glu im Hippocampus kann ein früher Hinweis auf Alzheimer sein. Auch wurde herausgefunden, dass die Kontrolle von Abweichungen an Dehydroepiandrosteron (DHEA) im Blut in Antwort auf oxidativen Stress ein nützlicher Test sein kann: Patienten mit MCI zeigten keine DHEA-Änderung, wohingegen die gesunden Kontrollen diese zeigten. Vor kurzem wurde ein miRNA (microRNA) Diagnosetest aus dem Serum vorgeschlagen (H. Geekiyanage, G.A. Jicha, P.T. Nelson, und C. Chan; Blood serum miRNA: Noninvasive biomarkers for Alzheimer's disease, Exp Neurol. 2012; 235(2): 491-496. doi:10.1016/j.expneurol.2011.11.026).

Hui Dong et al.; Serum MicroRNA Profiles Serve as Novel Biomarkers for the Diagnosis of Alzheimer's Disease, Disease Markers 2015, 14(1): 27, http://dx.doi.org/10.1155/2015/625659, beschreibt die Identifizierung und Validierung des Potentials von zirkulierenden miRNAs als Biomarker für Alzheimer.

EP 2 733 220 A1 betrifft miRNA Marker die insbesondere nützlich sind für die Diagnose von neuronalen Erkrankungen wie Alzheimer.

In der WO 2013/003350 A2 ist ein Verfahren zur Diagnose von Alzheimer beschrieben, bei der das Niveau von zumindest einer miRNA in einer Probe eines Patienten bestimmt wird.

Zudem sind der WO 2009/009457 A1 Verfahren zur Diagnose und/oder Prognose von Alzheimer in Patienten durch Messung der Menge von einer oder mehr mikro-RNAs in einer biologischen Probe offenbart.

In der WO 2011/057003 A2 sind Verfahren zur Detektion neuronaler Pathologien unter Verwendung einer quantitativen Analyse in Körperflüssigkeiten von synaptischen und/oder Neuritkleiner RNA sowie die Anwendung davon zur frühen Diagnose und dem Überwachen von neurodegenerativen Erkrankungen beschrieben.

Michel J. Weber, New human and mouse microRNA genes found by homology search, FEBS Journal 2005, 272(1), S. 59-73 offenbart die Bestimmung von miRNAs durch systematische orthologe Interspezies-Bestimmung von miRNA Präkursoren.

Die WO 2013/118066 A1 betrifft die Verwendung des miRNA-Expressionsprofils und der dadurch regulierten Target-Gene für die Diagnose, Prognose und die Verwendung von miR-199a-5p Inhibitoren für die Behandlung fibroproliferativer Erkrankungen.

Jedoch bleibt die sichere und frühe Diagnose von Alzheimer (AD) auf der Basis nicht-invasiver molekularer Biomarker eine Herausforderung. Zur Lösung der Aufgabe der vorliegenden Erfindung schlagen die Erfinder die Erstellung eines Expresssionsprofils einer Kombination von nicht-kodierenden microRNAs (miRNAs) zur Diagnose von Alzheimer aus Blutproben, insbesondere Blutzellen, vor.

Die Erfinder haben herausgefunden, dass mithilfe einer Änderung im Expressionsprofil von bestimmten miRNAs im Vergleich zu gesunden Patienten eine Diagnose von Alzheimer möglich ist.

Gemäß einem ersten Aspekt betrifft die Erfindung ein Verfahren zur Diagnose von Alzheimer in einem Patienten, umfassend: Bereitstellen einer Blutprobe eines Patienten, Erstellen eines Expressionsprofils von miRNAs aus der Blutprobe, und
Vergleich des Expressionsniveaus einer miRNA, die eine Sequenz umfasst, die ausgewählt ist aus hsa-miR-345-5p, und/oder die eine Sequenz umfasst, die von diesen Sequenzen um jeweils bis zu 3 Basen, bevorzugt bis zu 2 Basen, weiter bevorzugt 1 Base abweicht, mit einem Referenzniveau, wobei der Vergleich die Diagnose einer Erkrankung mit Alzheimer ermöglicht.

Ein weiterer Aspekt der Erfindung betrifft die Verwendung einer miRNA, die eine Sequenz umfasst, die ausgewählt ist aus hsa-miR-345-5p, und/oder die eine Sequenz umfasst, die von diesen Sequenzen um jeweils bis zu 3 Basen, bevorzugt bis zu 2 Basen, weiter bevorzugt 1 Base abweicht, als Marker für die Diagnose von Alzheimer.

Weitere Aspekte der vorliegenden Erfindung sind den abhängigen Ansprüchen und der detaillierten Beschreibung zu entnehmen.

Sofern nicht anderweitig definiert haben hierin verwendete technische und wissenschaftliche Begriffe dieselbe Bedeutung, wie sie im Allgemeinen von einem Fachmann im Gebiet der Erfindung verstanden wird.

MicroRNAs bzw. miRNAS bzw. micro-Ribonukleinsäuren sind eine Klasse von kurzen, nichtkodierenden RNAs, die beispielsweise eine Rolle bei der Genexpression spielen. Eine miRNA ist hierbei ein Polynucleotid mit einer festen Anzahl von Basen, beispielsweise von weniger als 500, weniger als 200, weniger als 100, weniger als 50 oder weniger als 30, aber beispielsweise mehr als 5, 10 oder 14 Basen, und einer definierten Sequenz. Aus dieser kann beispielsweise auch cDNA gewonnen werden, so dass aus den miRNAs im erfindungsgemäßen Verfahren auch cDNAs hergestellt werden können, welche dann ebenfalls mit cDNA-Niveaus verglichen werden können, die aus miRNAs von Patienten gewonnen werden, welche keine MS, insbesondere keine RRMS, aufweisen.

Im Folgenden wie auch zuvor beziehen sich die Verweise auf bestimmte miRNAs auf die Sequenzen, wie sie der Datenbank Mirbase (http://mirbase.org/), insbesondere Mirbase20 - also der Version 20 von Mirbase, entnommen werden können.

Des Weiteren sind Sequenzenauch der folgenden Tabelle 1 zu entnehmen. In Tabelle 1 sind hierbei die miRNAs in der reifen Form (mature Form) angegeben, welche von der Haarnadelform (stem loop form) abweichen kann.

**Tabelle 1: Sequenzen der miRNAs**

| **Reife Form** | **Sequenz der reifen Form** | **SEQ ID NO:** |
|---|---|---|
| | | |
| hsa-miR-345-5p | gcugacuccuaguccagggcuc | 1 |
| hsa-miR-5006-3p | uuucccuuuccauccuggcag | 2 |
| hsa-miR-7848-3p | cuacccucggucugcuuaccaca | 3 |
| hsa-miR-6817-3p | ucucucugacuccauggca | 4 |
| hsa-miR-361-5p | uuaucagaaucuccagggguac | 5 |
| hsa-miR-3157-3p | cugcccuagucuagcugaagcu | 6 |
| hsa-miR-4482-3p | uuucuauuucucaguggggcuc | 7 |
| hsa-miR-28-3p | cacuagauugugagcuccugga | 8 |
| hsa-miR-151a-3p | cuagacugaagcuccuugagg | 9 |
| hsa-miR-1468-5p | cuccguuugccuguuucgcug | 10 |
| hsa-miR-532-5p | caugccuugaguguaggaccgu | 11 |
| hsa-miR-17-3p | acugcagugaaggcacuuguag | 12 |
| hsa-miR-30a-3p | cuuucagucggauguuugcagc | 13 |

Die vorliegende Erfindung betrifft in einem ersten Aspekt ein Verfahren zur Diagnose von Alzheimer in einem Patienten, umfassend:
Bereitstellen einer Blutprobe eines Patienten,
Erstellen eines Expressionsprofils von miRNAs aus der Blutprobe, und
Vergleich des Expressionsniveaus einer miRNA, die eine Sequenz umfasst, die ausgewählt ist aus hsa-miR-345-5p, und/oder die eine Sequenz umfasst, die von dieser Sequenz um jeweils bis zu 3 Basen, bevorzugt bis zu 2 Basen, weiter bevorzugt 1 Base abweicht, mit einem Referenzniveau, wobei der Vergleich die Diagnose einer Erkrankung mit Alzheimer ermöglicht.

Beschrieben ist ein Verfahren zur Diagnose von Alzheimer in einem Patienten, umfassend:
Bereitstellen einer Blutprobe eines Patienten,
Erstellen eines Expressionsprofils von miRNAs aus der Blutprobe, und
Vergleich des Expressionsniveaus mindestens einer miRNA, die eine Sequenz umfasst, die ausgewählt ist aus der Gruppe, bestehend aus hsa-miR-345-5p, hsa-miR-5006-3p, hsa-miR-7848-3p, hsa-miR-6817-3p, hsa-miR-361-5p, hsa-miR-3157-3p, und hsa-miR-4482-3p, bevorzugt hsa-miR-345-5p, hsa-miR-5006-3p, hsa-miR-361-5p, und hsa-miR-3157-3p, und/oder die eine Sequenz umfasst, die von diesen Sequenzen um jeweils bis zu 3 Basen, bevorzugt bis zu 2 Basen, weiter bevorzugt 1 Base abweicht, mit einem Referenzniveau, wobei der Vergleich die Diagnose einer Erkrankung mit Alzheimer ermöglicht.

Das Expressionsprofil ist hierbei ein Profil der Expression von miRNAs, beispielsweise 1, 2, 3, 4, 5, 6, 7, 8, 9, 10, 11, 12, 13, 14, 15, 16, 17, 18, 19, 20, 30, 40, 50, 60, 70, 80, 90, 100 oder mehr miRNAs, in der Probe, gemäß bestimmten Ausführungsformen in quantitativer Form, während das Expressionsniveau die jeweilige Menge einer exprimierten miRNA angibt.

Das Bereitstellen der Blutprobe ist hierbei nicht besonders beschränkt, ist insbesondere jedoch nichtinvasiv, sondern es wird eine bereits genommene Blutprobe verwendet. Gemäß bestimmten Ausführungsformen kann auch die Entnahme und/oder Aufbereitung einer Blutprobe umfasst sein. Eine Blutprobe umfasst hierbei eine Vollblutprobe wie auch Fraktionen von Blutproben wie etwa das Plasma, Serum, etc., oder auch Zellen in der Blutprobe wie mononukleäre Zellen des peripheren Blutes. Gemäß bestimmten Ausführungsformen umfasst die Blutprobe Blutkörperchen oder besteht aus Blutkörperchen.

Der Patient, von dem die Blutprobe bereitgestellt wird, ist ebenfalls nicht besonders beschränkt, und kann beispielsweise Wirbeltiere, insbesondere Säugetiere umfassen, ist jedoch gemäß bestimmten Ausführungsformen ein Mensch. Insbesondere wird das vorliegende Verfahren gemäß bestimmten Ausführungsformen bei Patienten angewendet, z.B. Menschen, bei denen ein Verdacht auf eine Erkrankung mit Alzheimer besteht und/oder bei denen eine leichte kognitive Beeinträchtigung (MCI) diagnostiziert wurde.

Die Sequenzen, die im erfindungsgemäßen Verfahren zur Anwendung kommen, können hierbei insbesondere bei einer Veränderung des Expressionsniveaus einer oder mehrerer der miRNAs, die eine entsprechende Sequenz umfassen, zur Anwendung kommen.

Auch ist das Verfahren zum Erstellen eines Expressionsprofils von miRNAs nicht besonders beschränkt und kann hierbei beispielsweise gängige mikrobiologische Verfahren zur Bestimmung von Nukleinsäuren umfassen, wobei hier zum Vergleich mit dem Referenzniveau gemäß bestimmten Ausführungsformen ein semiquantitatives oder quantitatives, insbesondere quantitatives Verfahren zur Anwendung kommt. Gemäß bestimmten Ausführungsformen umfasst das Erstellen des Expressionsprofils von miRNAs mindestens ein Verfahren, dass ausgewählt ist aus der Gruppe, umfassend eine Nukleinsäurehybridisierung, eine Nukleinsäureamplifikation, eine Polymeraseextension, eine Sequenzierung, eine Massenspektrometrie, und jegliche Kombination davon.

Eine Nukleinsäurehybridisierung kann hierbei die Verwendung von Arrays, z.B. Mikroarrays, und/oder eine *in situ* Hybridisierung umfassen. Ein Array kann dazu beispielsweise die Verwendung komplementärer Sequenzen zu den bestimmten Sequenzen, also miRNAs, die eine Sequenz umfassen, die ausgewählt ist aus der Gruppe, bestehend aus hsa-miR-345-5p, hsa-miR-5006-3p, hsa-miR-7848-3p, hsa-miR-6817-3p, hsa-miR-361-5p, hsa-miR-3157-3p, und hsa-miR-4482-3p, bevorzugt hsa-miR-345-5p, hsa-miR-5006-3p, hsa-miR-361-5p, und hsa-miR-3157-3p, und zudem gemäß bestimmten Ausführungsformen zusätzlich miRNAs, die eine Sequenz umfassen, die ausgewählt ist aus der Gruppe, bestehend aus hsa-miR-28-3p, hsa-miR-151a-3p, hsa-miR-1468-5p, hsa-miR-532-5p, hsa-miR-17-3p, und hsa-miR-30a, und/oder die eine Sequenz umfassen, die von diesen Sequenzen um jeweils bis zu 3 Basen, bevorzugt bis zu 2 Basen, weiter bevorzugt 1 Base abweicht, umfassen, welche beispielsweise geeignet auf einen Träger angebracht werden können. Für eine Quantifizierung kann dann beispielsweise eine Markierung der miRNAs der Probe mit Markern wie Fluoreszenzmarkern oder Radionukleotidmarkern erfolgen.

Alternativ oder zusätzlich kann auch eine Polymerase-Kettenreaktion (PCR, polymerase chain reaction), bevorzugt eine quantitative PCR (qPCR), insbesondere eine quantitative Echtzeit-PCR (qRT-PCR, quantitative real-time PCR) auf geeignete Weise durchgeführt werden.

Auch sind Sequenzierungsmethoden wie Next Generation Sequenzing und/oder Massenspektrometrie, insbesondere quantitative Massenspektrometrie, denkbar.

Der Vergleich mit einem Referenzniveau im erfindungsgemäßen Verfahren ist nicht besonders beschränkt und kann hierbei insbesondere einen Vergleich mit der Probe eines gesunden Individuums und/oder eines Individuums mit einer MCI-Diagnose, bei dem kein Alzheimer diagnostiziert wird, umfassen. Das Verfahren erlaubt also nicht nur eine Diagnose, sondern auch ein Stadienbestimmung (Staging). Durch solch einen Vergleich kann beispielsweise die Diagnose einer Erkrankung mit Alzheimer ermöglicht werden. Ein Vergleich kann hierbei beispielsweise anhand mathematischer Ansätze wie Korrelationen, anhand statistischer Verfahren, anhand Wahrscheinlichkeitstheorien, anhand informationstheoretischer Ansätze oder Kombinationen davon stattfinden.

Für eine verbesserte Aussage ist hierbei ein statistischer Vergleich mit einer Vielzahl von Proben von gesunden und an Alzheimer erkrankten Patienten vorteilhaft, wobei solche Daten auch beispielsweise einer geeigneten Datenbank entnommen werden können und/oder anhand einer daraus entwickelten mathematischen Funktion bzw. eines Algorithmus bestimmt werden können. Das statistische Verfahren ist hierbei nicht besonders beschränkt und kann beispielsweise auch computergestützt durchgeführt werden. Beispielsweise kann hierbei ein t-Test, ein Wilcoxon-Mann-Whitney-Test, die Bestimmung der Fläche unter der Kurve (AUC, area under curve), etc. zur Anwendung kommen.

Gemäß bestimmten Ausführungsformen wird das Referenzniveau aus einer Vielzahl von Proben von Patienten erhalten, von denen eine erste Gruppe mit AD diagnostiziert wurde, und eine zweite Gruppe keine AD-Diagnose aufweist, also in dieser Hinsicht gesund ist, wobei bevorzugt die Gruppen gemäß Alter und Geschlecht angepasst sind, und wobei das Referenzniveau aus der Gruppe der gesunden Patienten bestimmt wird. Für eine weiterreichende Aussage können gemäß bestimmten Ausführungsformen auch noch Kontrollgruppen mit einer Diagnose von MCI und/oder MS herangezogen werden. Gemäß bestimmten Ausführungsformen wird hierbei ein Referenzniveau aus derselben Art von Probe bestimmt wie die zu bestimmende Probe.

Als Abweichung von der Sequenz ist hierbei eine Variation in der Sequenz, z.B. eine Addition, eine Insertion, eine Deletion, oder ein Austausch einer Base in der Sequenz im Vergleich zur ursprünglichen Sequenz, welche beispielsweise in Tabelle 1 angegeben ist, zu verstehen, insbesondere eine Deletion und/oder ein Einzelbasenaustausch. So kann beispielsweise eine Deletion von 1 oder 2 Basen an einem und/oder beiden Enden der Sequenz stattfinden, wobei maximal 3 Basen, bevorzugt maximal 2 Basen, weiter bevorzugt 1 Base deletiert werden und insbesondere keine Base deletiert wird. Auch ist zusätzlich dazu oder alternativ eine Änderung von 1 oder mehr Basen innerhalb der Sequenz möglich, wobei wiederum maximal eine Änderung um 3 Basen, bevorzugt 2 Basen, weiter bevorzugt 1 Base und insbesondere bevorzugt keiner Base im Vergleich zu den Sequenzen von hsa-miR-345-5p, hsa-miR-5006-3p, hsa-miR-7848-3p, hsa-miR-6817-3p, hsa-miR-361-5p, hsa-miR-3157-3p, und hsa-miR-4482-3p, bevorzugt hsa-miR-345-5p, hsa-miR-5006-3p, hsa-miR-361-5p, und hsa-miR-3157-3p, und/oder hsa-miR-28-3p, hsa-miR-151a-3p, hsa-miR-1468-5p, hsa-miR-532-5p, hsa-miR-17-3p, und hsa-miR-30a stattfindet.

Gemäß bestimmten Ausführungsformen findet also ein Vergleich des Expressionsniveaus mindestens einer miRNA, die eine Sequenz umfasst, die ausgewählt ist aus hsa-miR-345-5p, mit einem Referenzniveau statt.

Beschrieben ist ein Vergleich des Expressionsniveaus mindestens einer miRNA, die eine Sequenz umfasst, die ausgewählt ist aus der Gruppe, bestehend aus hsa-miR-345-5p, hsa-miR-5006-3p, hsa-miR-7848-3p, hsa-miR-6817-3p, hsa-miR-361-5p, hsa-miR-3157-3p, und hsa-miR-4482-3p, bevorzugt hsa-miR-345-5p, hsa-miR-5006-3p, hsa-miR-361-5p, und hsa-miR-3157-3p, mit einem Referenzniveau.

Es findet mindestens ein Vergleich mit einer Sequenz umfassend die miRNA hsa-miR-345-5p und/oder eine Sequenz, die von dieser Sequenz, also der Sequenz der miRNA, um bis zu 3 Basen, bevorzugt bis zu 2 Basen, weiter bevorzugt 1 Base abweicht, statt. Beschrieben ist mindestens ein Vergleich mit einer Sequenz umfassend die miRNA hsa-miR-5006-3p und/oder eine Sequenz, die von dieser Sequenz um bis zu 3 Basen, bevorzugt bis zu 2 Basen, weiter bevorzugt 1 Base abweicht. Beschrieben ist mindestens ein Vergleich mit einer Sequenz umfassend die miRNA hsa-miR-7848-3p und/oder eine Sequenz, die von dieser Sequenz um bis zu 3 Basen, bevorzugt bis zu 2 Basen, weiter bevorzugt 1 Base abweicht. Beschrieben ist mindestens ein Vergleich mit einer Sequenz umfassend die miRNA hsa-miR-6817-3p und/oder eine Sequenz, die von dieser Sequenz um bis zu 3 Basen, bevorzugt bis zu 2 Basen, weiter bevorzugt 1 Base abweicht. Beschrieben ist mindestens ein Vergleich mit einer Sequenz umfassend die miRNA hsa-miR-361-5p und/oder eine Sequenz, die von dieser Sequenz um bis zu 3 Basen, bevorzugt bis zu 2 Basen, weiter bevorzugt 1 Base abweicht. Beschrieben ist mindestens ein Vergleich mit einer Sequenz umfassend die miRNA hsa-miR-3157-3p und/oder eine Sequenz, die von dieser Sequenz um bis zu 3 Basen, bevorzugt bis zu 2 Basen, weiter bevorzugt 1 Base abweicht. Beschrieben ist mindestens ein Vergleich mit einer Sequenz umfassend die miRNA hsa-miR-4482-3p und/oder eine Sequenz, die von dieser Sequenz um bis zu 3 Basen, bevorzugt bis zu 2 Basen, weiter bevorzugt 1 Base abweicht.

Gemäß bestimmten Ausführungsformen findet mindestens ein Vergleich mit einer Sequenz umfassend die miRNA hsa-miR-345-5p statt. Beschrieben ist mindestens ein Vergleich mit einer Sequenz umfassend die miRNA hsa-miR-5006-3p. Beschrieben ist mindestens ein Vergleich mit einer Sequenz umfassend die miRNA hsa-miR-7848-3p. Beschrieben ist mindestens ein Vergleich mit einer Sequenz umfassend die miRNA hsa-miR-6817-3p. Beschrieben ist mindestens ein Vergleich mit einer Sequenz umfassend die miRNA hsa-miR-361-5p. Beschrieben ist mindestens ein Vergleich mit einer Sequenz umfassend die miRNA hsa-miR-3157-3p. Beschrieben ist mindestens ein Vergleich mit einer Sequenz umfassend die miRNA hsa-miR-4482-3p.

Gemäß bestimmten Ausführungsformen wird zudem das Expressionsniveau mindestens einer miRNA, die eine Sequenz umfasst, die ausgewählt ist aus der Gruppe, bestehend aus hsa-miR-5006-3p, hsa-miR-7848-3p, hsa-miR-6817-3p, hsa-miR-361-5p, hsa-miR-3157-3p, hsa-miR-4482-3p, hsa-miR-28-3p, hsa-miR-151a-3p, hsa-miR-1468-5p, hsa-miR-532-5p, hsa-miR-17-3p, und hsa-miR-30a-3p, und/oder die eine Sequenz umfasst, die von diesen Sequenzen um jeweils bis zu 3 Basen, bevorzugt bis zu 2 Basen, weiter bevorzugt 1 Base abweicht, mit einem Referenzniveau verglichen, wobei der Vergleich die Diagnose einer Erkrankung mit Alzheimer ermöglicht.

Gemäß bestimmten Ausführungsformen wird zudem das Expressionsniveau mindestens einer miRNA, die eine Sequenz umfasst, die ausgewählt ist aus der Gruppe, bestehend aus hsa-miR-28-3p, hsa-miR-151a-3p, hsa-miR-1468-5p, hsa-miR-532-5p, hsa-miR-17-3p, und hsa-miR-30a-3p, und/oder die eine Sequenz umfasst, die von diesen Sequenzen um jeweils bis zu 3 Basen, bevorzugt bis zu 2 Basen, weiter bevorzugt 1 Base abweicht, mit einem Referenzniveau verglichen, wobei der Vergleich die Diagnose einer Erkrankung mit Alzheimer ermöglicht.

Gemäß bestimmten Ausführungsformen wird in mindestens 2, 3, 4, 5, 6, 7, 8, 9, 10, 11, 12 oder 13 miRNAs, die eine Sequenz umfassen, die ausgewählt ist aus der Gruppe, bestehend aus hsa-miR-345-5p, hsa-miR-5006-3p, hsa-miR-7848-3p, hsa-miR-6817-3p, hsa-miR-361-5p, hsa-miR-3157-3p, hsa-miR-4482-3p, hsa-miR-28-3p, hsa-miR-151a-3p, hsa-miR-1468-5p, hsa-miR-532-5p, hsa-miR-17-3p, und hsa-miR-30a-3p, und/oder die eine Sequenz umfassen, die von diesen Sequenzen um jeweils bis zu 3 Basen, bevorzugt bis zu 2 Basen, weiter bevorzugt 1 Base abweicht, eine Änderung im Expressionsniveau im Vergleich zu einem Referenzniveau beobachtet, wobei in einer miRNA, die eine Sequenz umfasst, die ausgewählt ist aus hsa-miR-345-5p, und/oder die eine Sequenz umfasst, die von dieser Sequenz um jeweils bis zu 3 Basen, bevorzugt bis zu 2 Basen, weiter bevorzugt 1 Base abweicht, eine Änderung im Expressionsniveau im Vergleich zu einem Referenzniveau beobachtet wird. Gemäß bestimmten Ausführungsformen wird in mindestens 2, 3, 4, 5, 6, 7, 8, 9, 10, 11, 12 oder 13 miRNAs, die eine Sequenz umfassen, die ausgewählt ist aus der Gruppe, bestehend aus hsa-miR-345-5p, hsa-miR-5006-3p, hsa-miR-7848-3p, hsa-miR-6817-3p, hsa-miR-361-5p, hsa-miR-3157-3p, hsa-miR-4482-3p, hsa-miR-28-3p, hsa-miR-151a-3p, hsa-miR-1468-5p, hsa-miR-532-5p, hsa-miR-17-3p, und hsa-miR-30a-3p, eine Änderung im Expressionsniveau im Vergleich zu einem Referenzniveau beobachtet, wobei in einer miRNA, die eine Sequenz umfasst, die ausgewählt ist aus hsa-miR-345-5p, eine Änderung im Expressionsniveau im Vergleich zu einem Referenzniveau beobachtet wird.

Gemäß bestimmten Ausführungsformen wird in mindestens 2, 3, 4, 5, 6, oder 7 miRNAs, die eine Sequenz umfassen, die ausgewählt ist aus der Gruppe, bestehend aus hsa-miR-345-5p, hsa-miR-5006-3p, hsa-miR-7848-3p, hsa-miR-6817-3p, hsa-miR-361-5p, hsa-miR-3157-3p, und hsa-miR-4482-3p, bevorzugt hsa-miR-345-5p, hsa-miR-5006-3p, hsa-miR-361-5p, und hsa-miR-3157-3p, und/oder die eine Sequenz umfassen, die von diesen Sequenzen um jeweils bis zu 3 Basen, bevorzugt bis zu 2 Basen, weiter bevorzugt 1 Base abweicht, eine Änderung im Expressionsniveau im Vergleich zu einem Referenzniveau beobachtet, wobei in einer miRNA, die eine Sequenz umfasst, die ausgewählt ist aus hsa-miR-345-5p, und/oder die eine Sequenz umfasst, die von dieser Sequenz um jeweils bis zu 3 Basen, bevorzugt bis zu 2 Basen, weiter bevorzugt 1 Base abweicht, eine Änderung im Expressionsniveau im Vergleich zu einem Referenzniveau beobachtet wird. Gemäß bestimmten Ausführungsformen wird in mindestens 2, 3, 4, 5, 6, oder 7 miRNAs, die eine Sequenz umfassen, die ausgewählt ist aus der Gruppe, bestehend aus hsa-miR-345-5p, hsa-miR-5006-3p, hsa-miR-7848-3p, hsa-miR-6817-3p, hsa-miR-361-5p, hsa-miR-3157-3p, und hsa-miR-4482-3p, bevorzugt hsa-miR-345-5p, hsa-miR-5006-3p, hsa-miR-361-5p, und hsa-miR-3157-3p, eine Änderung im Expressionsniveau im Vergleich zu einem Referenzniveau beobachtet, wobei in einer miRNA, die eine Sequenz umfasst, die ausgewählt ist aus hsa-miR-345-5p, eine Änderung im Expressionsniveau im Vergleich zu einem Referenzniveau beobachtet wird.

Bei einer Änderung im Expressionsniveau von mehr als einer miRNA kann hierbei das Signifikanzniveau für eine Diagnose mit einer Alzheimer-Erkrankung außerordentlich gesteigert werden.

Die miRNA ist ausgewählt aus der Gruppe, bestehend aus hsa-miR-345, und/oder mit einer Sequenz, die von dieser Sequenz um bis zu 3 Basen, bevorzugt bis zu 2 Basen, weiter bevorzugt 1 Base abweicht. Gemäß bestimmten Ausführungsformen ist die miRNA hsa-miR-345. Gemäß bestimmten Ausführungsformen ist die weitere miRNA, die zudem bestimmt wird zu der miRNA, die hsa-miR-345-5p ist, und/oder die eine Sequenz umfasst, die von diesen Sequenzen um jeweils bis zu 3 Basen, bevorzugt bis zu 2 Basen, weiter bevorzugt 1 Base abweicht, mindestens eine, die ausgewählt ist aus der Gruppe, bestehend aus hsa-miR-5006, hsa-miR-7848, hsa-miR-6817, hsa-miR-361, hsa-miR-3157, hsa-miR-4482, hsa-miR-28, hsa-miR-151a, hsa-miR-1468, hsa-miR-532, hsa-miR-17, und hsa-miR-30a, und/oder mit einer Sequenz, die von dieser Sequenz um bis zu 3 Basen, bevorzugt bis zu 2 Basen, weiter bevorzugt 1 Base abweicht. Gemäß bestimmten Ausführungsformen ist die weitere miRNA, die zudem bestimmt wird zu der miRNA, die hsa-miR-345-5p ist, und/oder die eine Sequenz umfasst, die von diesen Sequenzen um jeweils bis zu 3 Basen, bevorzugt bis zu 2 Basen, weiter bevorzugt 1 Base abweicht, mindestens eine, die ausgewählt ist aus der Gruppe, bestehend aus hsa-miR-5006, hsa-miR-7848, hsa-miR-6817, hsa-miR-361, hsa-miR-3157, hsa-miR-4482, hsa-miR-28, hsa-miR-151a, hsa-miR-1468, hsa-miR-532, hsa-miR-17, und hsa-miR-30a. Gemäß bestimmten Ausführungsformen wird also zudem das Expressionsniveau mindestens einer miRNA bestimmt, die ausgewählt ist aus der Gruppe, bestehend aus hsa-miR-5006, hsa-miR-7848, hsa-miR-6817, hsa-miR-361, hsa-miR-3157, hsa-miR-4482, hsa-miR-28, hsa-miR-151a, hsa-miR-1468, hsa-miR-532, hsa-miR-17 und hsa-miR-30a.

Diese Ausführungsformen der miRNA entsprechen hierbei der Stem-Loop Form bzw. Haarnadelform, welche mit der detektierten reifen Form wie in Tabelle 2 (sowohl für die miRNAs der erfindungsgemäßen Verwendung wie auch für die weiteren, im erfindungsgemäßen Verfahren anwendbaren miRNAs) gezeigt korreliert, und welche beispielsweise in den Proben als solche enthalten sein können. Entsprechend kann der Vergleich anstelle mit der jeweiligen reifen Form auch mit der entsprechenden Stem-Loop Form erfolgen.

**Tabelle 2: Korrelation zwischen Stem-Loop Form und reifen Form**

| **Stem-Loop form** | **SEQ ID NO:** | **Reife Form** | **Sequenz der reifen Form** |
|---|---|---|---|
| | | | |
| hsa-miR-345 | 14 | hsa-miR-345-5p | gcugacuccuaguccagggcuc |
| hsa-miR-5006 | 15 | hsa-miR-5006-3p | uuucccuuuccauccuggcag |
| hsa-miR-7848 | 16 | hsa-miR-7848-3p | cuacccucggucugcuuaccaca |
| hsa-miR-6817 | 17 | hsa-miR-6817-3p | ucucucugacuccauggca |
| hsa-miR-361 | 18 | hsa-miR-361-5p | uuaucagaaucuccagggguac |
| hsa-miR-3157 | 19 | hsa-miR-3157-3p | cugcccuagucuagcugaagcu |
| hsa-miR-4482 | 20 | hsa-miR-4482-3p | uuucuauuucucaguggggcuc |
| hsa-miR-28 | 21 | hsa-miR-28-3p | cacuagauugugagcuccugga |
| hsa-miR-151a | 22 | hsa-miR-151a-3p | cuagacugaagcuccuugagg |
| hsa-miR-1468 | 23 | hsa-miR-1468-5p | cuccguuugccuguuucgcug |
| hsa-miR-532 | 24 | hsa-miR-532-5p | caugccuugaguguaggaccgu |
| hsa-miR-17 | 25 | hsa-miR-17-3p | acugcagugaaggcacuuguag |
| hsa-miR-30a | 26 | hsa-miR-30a-3p | cuuucagucggauguuugcagc |

Die Nukleinsäuresequenzen der Stem-Loop Formen gemäß Tabelle 2 sind wie folgt:

Gemäß bestimmten Ausführungsformen wird in mindestens 2, 3, 4, 5, 6, oder 7, miRNAs, die eine Sequenz umfassen, die ausgewählt ist aus der Gruppe, bestehend aus hsa-miR-345, hsa-miR-5006, hsa-miR-7848, hsa-miR-6817, hsa-miR-361, hsa-miR-3157 und hsa-miR-4482, und/oder die eine Sequenz umfassen, die von diesen Sequenzen um jeweils bis zu 3 Basen, bevorzugt bis zu 2 Basen, weiter bevorzugt 1 Base abweicht, eine Änderung im Expressionsniveau im Vergleich zu einem Referenzniveau beobachtet, wobei in einer miRNA, die eine Sequenz umfasst, die ausgewählt ist aus hsa-miR-345-5p, und/oder die eine Sequenz umfasst, die von dieser Sequenz um jeweils bis zu 3 Basen, bevorzugt bis zu 2 Basen, weiter bevorzugt 1 Base abweicht, eine Änderung im Expressionsniveau im Vergleich zu einem Referenzniveau beobachtet wird. Gemäß bestimmten Ausführungsformen wird in mindestens 2, 3, 4, 5, 6, oder 7, miRNAs, die eine Sequenz umfassen, die ausgewählt ist aus der Gruppe, bestehend aus hsa-miR-345, hsa-miR-5006, hsa-miR-7848, hsa-miR-6817, hsa-miR-361, hsa-miR-3157 und hsa-miR-4482, eine Änderung im Expressionsniveau im Vergleich zu einem Referenzniveau beobachtet, wobei in einer miRNA, die eine Sequenz umfasst, die ausgewählt ist aus hsa-miR-345-5p, eine Änderung im Expressionsniveau im Vergleich zu einem Referenzniveau beobachtet wird.

Gemäß einem weiteren Aspekt betrifft die Erfindung die Verwendung einer miRNA, die eine Sequenz umfasst, die ausgewählt ist aus hsa-miR-345-5p, und/oder die eine Sequenz umfasst, die von dieser Sequenz um jeweils bis zu 3 Basen, bevorzugt bis zu 2 Basen, weiter bevorzugt 1 Base abweicht, als Marker für die Diagnose von Alzheimer.

Beschrieben ist die Verwendung mindestens einer miRNA, die eine Sequenz umfasst, die ausgewählt ist aus der Gruppe, bestehend aus hsa-miR-345-5p, hsa-miR-5006-3p, hsa-miR-7848-3p, hsa-miR-6817-3p, hsa-miR-361-5p, hsa-miR-3157-3p, und hsa-miR-4482-3p, bevorzugt hsa-miR-345-5p, hsa-miR-5006-3p, hsa-miR-361-5p, und hsa-miR-3157-3p, und/oder die eine Sequenz umfasst, die von diesen Sequenzen um jeweils bis zu 3 Basen, bevorzugt bis zu 2 Basen, weiter bevorzugt 1 Base abweicht, als Marker für die Diagnose von Alzheimer. Gemäß bestimmten Ausführungsformen wird eine miRNA, die eine Sequenz umfasst, bestehend aus hsa-miR-345-5p, verwendet. Beschrieben ist die Verwendung mindestens einer miRNA, die eine Sequenz umfasst, die ausgewählt ist aus der Gruppe, bestehend aus hsa-miR-345-5p, hsa-miR-5006-3p, hsa-miR-7848-3p, hsa-miR-6817-3p, hsa-miR-361-5p, hsa-miR-3157-3p, und hsa-miR-4482-3p, bevorzugt hsa-miR-345-5p, hsa-miR-5006-3p, hsa-miR-361-5p, und hsa-miR-3157-3p.

Beschrieben ist, dass hierbei die miRNA mindestens eine ist, die eine Sequenz aufweist, die ausgewählt ist aus der Gruppe, bestehend aus hsa-miR-345, hsa-miR-5006, hsa-miR-7848, hsa-miR-6817, hsa-miR-361, hsa-miR-3157 und hsa-miR-4482, bevorzugt hsa-miR-345, hsa-miR-5006, hsa-miR-361, und hsa-miR-3157, und/oder die eine Sequenz aufweist, die von diesen Sequenzen um jeweils bis zu 3 Basen, bevorzugt bis zu 2 Basen, weiter bevorzugt 1 Base abweicht. Beschrieben ist, dass hierbei die miRNA mindestens eine ist, die eine Sequenz aufweist, die ausgewählt ist aus der Gruppe, bestehend aus hsa-miR-345, hsa-miR-5006, hsa-miR-7848, hsa-miR-6817, hsa-miR-361, hsa-miR-3157 und hsa-miR-4482, bevorzugt hsa-miR-345, hsa-miR-5006, hsa-miR-361, und hsa-miR-3157.

Zudem beschrieben ist ein Kit zur Diagnose von Alzheimer in einer Blutprobe eines Patienten, umfassend:
Sonden und/oder Primer zur Detektion mindestens einer miRNA-Sequenz, die eine Sequenz umfasst, die ausgewählt ist aus der Gruppe, bestehend aus hsa-miR-345-5p, hsa-miR-5006-3p, hsa-miR-7848-3p, hsa-miR-6817-3p, hsa-miR-361-5p, hsa-miR-3157-3p, und hsa-miR-4482-3p, bevorzugt hsa-miR-345-5p, hsa-miR-5006-3p, hsa-miR-361-5p, und hsa-miR-3157-3p, und/oder die eine Sequenz umfasst, die von diesen Sequenzen um jeweils bis zu 3 Basen, bevorzugt bis zu 2 Basen, weiter bevorzugt 1 Base abweicht.

Das Kit kann Sonden und/oder Primer zur Detektion mindestens einer miRNA-Sequenz, die eine Sequenz umfasst, die ausgewählt ist aus der Gruppe, bestehend aus hsa-miR-345-5p, hsa-miR-5006-3p, hsa-miR-7848-3p, hsa-miR-6817-3p, hsa-miR-361-5p, hsa-miR-3157-3p, und hsa-miR-4482-3p, bevorzugt hsa-miR-345-5p, hsa-miR-5006-3p, hsa-miR-361-5p, und hsa-miR-3157-3p, umfassen.

Das Kit kann Sonden und/oder Primer zur Detektion mindestens einer miRNA-Sequenz, die eine Sequenz aufweist, die ausgewählt ist aus der Gruppe, bestehend aus hsa-miR-345, hsa-miR-5006, hsa-miR-7848, hsa-miR-6817, hsa-miR-361, hsa-miR-3157 und hsa-miR-4482, bevorzugt hsa-miR-345, hsa-miR-5006, hsa-miR-361, und hsa-miR-3157, und/oder die eine Sequenz aufweist, die von diesen Sequenzen um jeweils bis zu 3 Basen, bevorzugt bis zu 2 Basen, weiter bevorzugt 1 Base abweicht, umfassen.

Das Kit kann Sonden und/oder Primer zur Detektion mindestens einer miRNA-Sequenz, die eine Sequenz aufweist, die ausgewählt ist aus der Gruppe, bestehend aus hsa-miR-345, hsa-miR-5006, hsa-miR-7848, hsa-miR-6817, hsa-miR-361, hsa-miR-3157 und hsa-miR-4482 bevorzugt hsa-miR-345, hsa-miR-5006, hsa-miR-361, und hsa-miR-3157, umfassen.

Das Kit kann zudem Sonden und/oder Primer umfassen zur Detektion mindestens einer miRNA-Sequenz, die eine Sequenz umfasst, die ausgewählt ist aus der Gruppe, bestehend aus hsa-miR-28-3p, hsa-miR-151a-3p, hsa-miR-1468-5p, hsa-miR-532-5p, hsa-miR-17-3p, und hsa-miR-30a-3p, und/oder die eine Sequenz umfasst, die von diesen Sequenzen um jeweils bis zu 3 Basen, bevorzugt bis zu 2 Basen, weiter bevorzugt 1 Base abweicht, bevorzugt Sonden und/oder Primer zur Detektion mindestens einer miRNA-Sequenz, die eine Sequenz umfasst, die ausgewählt ist aus der Gruppe, bestehend aus hsa-miR-28-3p, hsa-miR-151a-3p, hsa-miR-1468-5p, hsa-miR-532-5p, hsa-miR-17-3p, und hsa-miR-30a-3p. Das Kit kann insbesondere zudem Sonden und/oder Primer umfassen zur Detektion mindestens einer miRNA-Sequenz, die eine Sequenz aufweist, die ausgewählt ist aus der Gruppe, bestehend aus hsa-miR-28, hsa-miR-151a, hsa-miR-1468, hsa-miR-532, hsa-miR-17 und hsa-miR-30a, und/oder die eine Sequenz aufweist, die von diesen Sequenzen um jeweils bis zu 3 Basen, bevorzugt bis zu 2 Basen, weiter bevorzugt 1 Base abweicht, bevorzugt Sonden und/oder Primer zur Detektion mindestens einer miRNA-Sequenz, die eine Sequenz aufweist, die ausgewählt ist aus der Gruppe, bestehend aus hsa-miR-28, hsa-miR-151a, hsa-miR-1468, hsa-miR-532, hsa-miR-17 und hsa-miR-30a.

Das Kit kann Sonden und/oder Primer zur Detektion von 2, 3, 4, 5, 6, 7, 8, 9, 10, 11, 12 oder 13 der obigen Sequenzen umfassen.

Die Sonden und/oder Primer sind hierbei nicht besonders beschränkt und können beispielsweise Oligomere umfassen, welche eine Nukleotidsequenz aufweisen, beispielsweise auch eine cDNA-Sequenz, welche komplementär zur Sequenz ist, die zu detektieren ist.

Daneben können auch weitere Sonden und/oder Primer als Kontollen vorgesehen sein, beispielsweise von weiteren miRNAs, bei denen keine Änderung des Expressionsprofils im Vergleich zu Patienten ohne AD-Erkrankung auftritt. Auch können Kontrollen für nicht-spezifisches Binden und/oder Hybridisieren vorgesehen sein.

Die Primer und/oder Sonden sowie ggf. Kontrollen können auf ein geeignetes Substrat aufgebracht sein.

Das Kit kann weiter Enzyme und/oder Reagenzien zur Durchführung einer RT-PCR, insbesondere qRT-PCR, umfassen, wobei diese nicht besonders beschränkt sind. Reagenzien können hierbei beispielsweise auch Marker, z.B. zur Fluoreszenzmarkierung und/oder Radionukleotid-Markierung, umfassen. Auch kann das Kit Reagenzien zur cDNA-Synthese aus den miRNAs vor der PCR, bevorzugt qPCR und/oder RT-PCR, insbesondere qRT-PCR, umfassen.

Die obigen Ausführungsformen, Ausgestaltungen und Weiterbildungen lassen sich, sofern sinnvoll, beliebig miteinander kombinieren. Weitere mögliche Ausgestaltungen, Weiterbildungen und Implementierungen der Erfindung umfassen auch nicht explizit genannte Kombinationen von zuvor oder im Folgenden bezüglich der Ausführungsbeispiele beschriebenen Merkmalen der Erfindung. Insbesondere wird der Fachmann auch Einzelaspekte als Verbesserungen oder Ergänzungen zu der jeweiligen Grundform der vorliegenden Erfindung hinzufügen.

Die Erfindung wird im Anschluss anhand von beispielhaften Ausführungsformen weiter verdeutlicht, welche die Erfindung jedoch nicht einschränken.

### Beispiel 1

Im Beispiel wurden Proben von Individuen mittels Hochdurchsatz-Sequenzierung (high throughput sequencing) als unbefangene Technologie untersucht. Es wurde eine blinde multizentrische Fall-Kontroll-Studie (multi-centric case-control study) mit 294 Individuen, welche 107 AD Patienten, 20 MCI Patienten 90 MS Patienten und 77 im Alter und Geschlecht angepasste Kontollen beinhalteten, durchgeführt. Die miRNome - also Gesamtheiten der microRNAs - aller Individuen wurden unter Verwendung von Next-Generation-Sequencing (NGS) auf übliche Weise untersucht. DIE AD-Patienten und Kontrollen teilten sich hierbei in eine erste Kohorte mit 54 AD Patienten und 22 Kontrollen aus den Vereinigten Staaten, die zunächst überprüft wurden, und eine zweite Replikations-Kohorte mit 53 AD Patienten und 55 Kontrollen eines deutschen Universitätsklinikums auf.

Bei der Durchführung des NGS mit den 294 Individuen wurden in etwa 6 Milliarden kurze RNA-Sequenzen als ausgelesene Werte (reads) erzeugt. Aus den Daten aus den USA und Deutschland wurden eine AD-Gruppe und eine Kontroll-Gruppe profiliert. Es wurden insgesamt 586 miRNAs im Blut gefunden. In der US-Kohorte waren 210 miRNAs in der Expression zwischen den AD- und Kontroll-Proben verändert, und in der deutschen Kohorte 135 miRNAs. In beiden Studien wurde ein signifikanter (p < 0,00001) Überlapp von 69 miRNAs gefunden. Von diesen stimmte die Expressionsrichtung von 96% in beiden Kohorten überein, und die Flächen unter der Kurve (AUC, area under curve) Werte zeigten eine hoch signifikante Korrelation (p < 10⁻¹⁶) von 0,92 (95% Konfidenzintervall (CI) von 0,87-0,95).

Die hierin gefundenen statistisch signifikanten miRNAs, welche zur Diagnose von Alzheimer herangezogen werden können, sind in Tabelle 3 gegeben.

Die miRNAs in der Signatur scheinen in der mitochondrialen Dysfunktion durch die potentiellen Ziel- bzw. Target-Gene PARL (p = 0,004) und SLC25A5 (p = 0,002) involviert zu sein.

**Tabelle 3: miRNAs mit signifikant unterschiedlichem Expressionsniveau in AD-Patienten im Vergleich zur Kontrollgruppe**

| **Reife Form** | **t-Test AD vs. Kontrolle** | **AUC AD vs. Kontrolle** |
|---|---|---|
| | | |
| hsa-miR-345-5p | 3,20E-04 | 0,33 |
| hsa-miR-5006-3p | 1,26E-04 | 0,36 |
| hsa-miR-7848-3p | 2,28E-03 | 0,40 |
| hsa-miR-6817-3p | 1,02E-03 | 0,42 |
| hsa-miR-361-5p | 1,25E-07 | 0,29 |
| hsa-miR-3157-3p | 1,14E-07 | 0,29 |
| hsa-miR-4482-3p | 3,46E-03 | 0,35 |
| hsa-miR-28-3p | 1,70E-10 | 0,24 |
| hsa-miR-151a-3p | 7,13E-11 | 0,25 |
| hsa-miR-1468-5p | 7,11E-08 | 0,32 |
| hsa-miR-532-5p | 6,94E-04 | 0,65 |
| hsa-miR-17-3p | 5,63E-08 | 0,78 |
| hsa-miR-30a-3p | 1,65E-08 | 0,24 |

Weitere gefundene relevante reife miRNA-Sequenzen für die beiden untersuchten Kohorten, die anhand eines Wilcoxon-Mann-Whitney-Tests bestimmt wurden, sind in Tabelle 4 mit den gefundenen Werten aus jeweils einem pro Kohorte durchgeführten Wilcoxon-Mann-Whitney-Test gezeigt.

**Tabelle 4: miRNAs mit unterschiedlichem Expressionsniveau in AD-Patienten im Vergleich zur Kontrollgruppe anhand der Ergebnisse von Wilcoxon-Mann-Whitney-Tests für die erste und zweite Kohorte**

| **miRNA** | **Reife Sequenz** | **SEQ ID NO:** | **Wilcoxon Mann Whitney Test (1.Kohorte)** | **Wilcoxon Mann Whitney Test (2.Kohorte)** |
|---|---|---|---|---|
| hsa-miR-17-3p | acugcagugaaggcacuuguag | 12 | 1,15E-05 | 0,00020271 |
| hsa-miR-30a-3p | cuuucagucggauguuugcagc | 13 | 0,0035552 | 4,14E-06 |
| hsa-miR-28-3p | cacuagauugugagcuccugga | 8 | 0,00038033 | 6,33E-06 |
| hsa-miR-30e-3p | cuuucagucggauguuuacagc | 27 | 0,0073513 | 2,34E-06 |
| hsa-miR-151a-3p | cuagacugaagcuccuugagg | 9 | 1,70E-07 | 0,0045649 |
| hsa-miR-4781-3p | aauguuggaauccucgcuagag | 28 | 2,44E-06 | 0,043509 |
| hsa-miR-4508 | gcggggcugggcgcgcg | 29 | 0,020063 | 0,0011682 |
| hsa-miR-361-5p | uuaucagaaucuccagggguac | 5 | 3,13E-05 | 0,00010372 |
| hsa-miR-3157-3p | cugcccuagucuagcugaagcu | 6 | 5,74E-06 | 0,0052611 |
| hsa-miR-33b-5p | gugcauugcuguugcauugc | 30 | 9,95E-05 | 0,0035992 |
| hsa-miR-16-2-3p | ccaauauuacugugcugcuuua | 31 | 0,027024 | 0,00018878 |
| hsa-miR-574-5p | ugagugugugugugugagugugu | 32 | 0,025881 | 0,0046907 |
| hsa-miR-5690 | ucagcuacuaccucuauuagg | 33 | 0,0023133 | 0,0067456 |
| hsa-miR-363-3p | aauugcacgguauccaucugua | 34 | 0,0043552 | 0,0017395 |
| hsa-miR-4746-5p | ccggucccaggagaaccugcaga | 35 | 0,018015 | 0,003912 |
| hsa-let-7b-5p | ugagguaguagguugugugguu | 36 | 0,029466 | 0,0060788 |
| hsa-miR-1468-5p | cuccguuugccuguuucgcug | 10 | 0,0041147 | 0,0019343 |
| hsa-miR-345-5p | gcugacuccuaguccagggcuc | 1 | 0,0012034 | 0,019022 |
| hsa-miR-378d | acuggacuuggagucagaaa | 37 | 0,0027945 | 0,00033694 |
| hsa-miR-378g | acugggcuuggagucagaag | 38 | 0,0031832 | 0,00021093 |
| hsa-miR-221-3p | agcuacauugucugcuggguuuc | 39 | 0,004188 | 0,0068757 |
| hsa-miR-378f | acuggacuuggagccagaag | 40 | 0,0024916 | 0,0023304 |
| hsa-miR-340-3p | uccgucucaguuacuuuauagc | 41 | 0,0002208 | 0,00063591 |
| hsa-let-7c-5p | ugagguaguagguuguaugguu | 42 | 8,00E-06 | 0,0047904 |
| hsa-miR-532-5p | caugccuugaguguaggaccgu | 11 | 0,0056523 | 0,0061427 |
| hsa-miR-3605-3p | ccuccguguuaccuguccucuag | 43 | 0,031272 | 8,42E-06 |
| hsa-miR-340-5p | uuauaaagcaaugagacugauu | 44 | 0,00092804 | 0,0023566 |
| hsa-miR-5006-3p | uuucccuuuccauccuggcag | 2 | 0,015025 | 0,034036 |
| hsa-miR-3909 | uguccucuagggccugcagucu | 45 | 0,00072755 | 0,0072214 |
| hsa-let-7d-3p | cuauacgaccugcugccuuucu | 46 | 0,014922 | 0,00060374 |
| hsa-miR-128-3p | ucacagugaaccggucucuuu | 47 | 1,04E-05 | 0,010414 |
| hsa-miR-330-5p | ucucugggccugugucuuaggc | 48 | 7,40E-05 | 0,00086506 |
| hsa-miR-548e-3p | aaaaacugagacuacuuuugca | 49 | 0,0090437 | 0,036586 |
| hsa-miR-548ad-5p | aaaaguaauugugguuuuug | 50 | 0,0047025 | 0,00040004 |
| hsa-miR-548d-5p | aaaaguaauugugguuuuugcc | 51 | 0,0047025 | 0,00040004 |
| hsa-miR-548ae-5p | aaaaguaauugugguuuuug | 52 | 0,0031776 | 0,0040292 |
| hsa-miR-548ay-5p | aaaaguaauugugguuuuugc | 53 | 0,0031776 | 0,0040292 |
| hsa-miR-107 | agcagcauuguacagggcuauca | 54 | 6,13E-05 | 0,00026867 |
| hsa-miR-106b-5p | uaaagugcugacagugcagau | 55 | 0,028274 | 0,0020005 |
| hsa-miR-548az-5p | caaaagugauugugguuuuugc | 56 | 0,0051462 | 0,0037591 |
| hsa-miR-103a-3p | agcagcauuguacagggcuauga | 57 | 0,00017315 | 0,00048883 |
| hsa-let-7d-5p | agagguaguagguugcauaguu | 58 | 0,014163 | 0,0079347 |
| hsa-miR-190a-5p | ugauauguuugauauauuaggu | 59 | 1,61E-05 | 0,0019051 |
| hsa-miR-3074-5p | guuccugcugaacugagccag | 60 | 0,0081401 | 0,018552 |
| hsa-miR-550a-3p | ugucuuacucccucaggcacau | 61 | 0,0066173 | 0,0034703 |
| hsa-miR-106a-5p | aaaagugcuuacagugcagguag | 62 | 0,014462 | 0,016999 |
| hsa-miR-598-3p | uacgucaucguugucaucguca | 63 | 0,0042481 | 0,0055876 |
| hsa-miR-1294 | ugugagguuggcauuguugucu | 64 | 1,88E-05 | 0,026805 |
| hsa-miR-5010-3p | uuuugugucucccauuccccag | 65 | 2,25E-05 | 0,037426 |
| hsa-let-7i-5p | ugagguaguaguuugugcuguu | 66 | 0,00012721 | 0,042682 |
| hsa-miR-17-5p | caaagugcuuacagugcagguag | 67 | 0,039237 | 0,033477 |
| hsa-miR-660-5p | uacccauugcauaucggaguug | 68 | 3,50E-06 | 0,011728 |
| hsa-miR-6754-3p | ucuucaccugccucugccugca | 69 | 0,018408 | 0,013335 |
| hsa-miR-101-3p | uacaguacugugauaacugaa | 70 | 1,06E-06 | 0,0019576 |
| hsa-let-7e-5p | ugagguaggagguuguauaguu | 71 | 8,31E-07 | 0,0034125 |
| hsa-miR-6842-3p | uuggcuggucucugcuccgcag | 72 | 0,0030116 | 0,0022308 |
| hsa-miR-328-3p | cuggcccucucugcccuuccgu | 73 | 0,0087011 | 0,0018858 |
| hsa-miR-1285-5p | gaucucacuuuguugcccagg | 74 | 6,09E-05 | 0,0048518 |
| hsa-let-7f-5p | ugagguaguagauuguauaguu | 75 | 4,82E-09 | 0,0028229 |
| hsa-let-7a-5p | ugagguaguagguuguauaguu | 76 | 1,51E-08 | 0,004414 |
| hsa-miR-301a-3p | cagugcaauaguauugucaaagc | 77 | 0,0018718 | 0,012808 |
| hsa-miR-3127-3p | uccccuucugcaggccugcugg | 78 | 2,04E-05 | 0,045572 |
| hsa-miR-20a-5p | uaaagugcuuauagugcagguag | 79 | 0,025138 | 0,010058 |
| hsa-miR-6783-3p | uuccugggcuucuccucuguag | 80 | 0,029479 | 0,0093665 |
| hsa-miR-3615 | ucucucggcuccucgcggcuc | 81 | 0,016317 | 0,046337 |
| hsa-miR-98-5p | ugagguaguaaguuguauuguu | 82 | 9,18E-07 | 0,020253 |
| hsa-miR-5001-3p | uucugccucuguccagguccuu | 83 | 2,24E-06 | 0,01183 |
| hsa-let-7g-5p | ugagguaguaguuuguacaguu | 84 | 1,81E-07 | 0,017748 |

### Beispiel 2

Zum Abschätzen der Spezifizität der in Beispiel 1 gefundenen miRNAs für Alzheimer wurde weiterhin deren Expressionswert mit denen von MCI- und MS-(Multiple Sklerose)Patienten, die wie in Beispiel 1 gewonnen wurden, verglichen. Die Ergebnisse hierzu sind in Tabelle 5 gezeigt.

**Tabelle 5: miRNAs mit signifikant unterschiedlichem Expressionsniveau in AD-Patienten im Vergleich zu MCI- und MS-Patienten**

| **Reife Form** | **t-Test AD vs. MCI** | **AUC AD vs. MCI** | **t-Test AD vs. MS** | **AUC AD vs. MS** |
|---|---|---|---|---|
| | | | | |
| hsa-miR-345-5p | 4,96E-02 | 0,38 | 6,83E-04 | 0,39 |
| hsa-miR-5006-3p | 2,88E-02 | 0,32 | 1,16E-02 | 0,37 |
| hsa-miR-7848-3p | 8,76E-01 | 0,44 | 1,81E-06 | 0,27 |
| hsa-miR-6817-3p | 5,03E-01 | 0,40 | 3,98E-10 | 0,28 |
| hsa-miR-361-5p | 3,81E-02 | 0,28 | 3,93E-04 | 0,36 |
| hsa-miR-3157-3p | 1,74E-02 | 0,29 | 5,17E-13 | 0,21 |
| hsa-miR-4482-3p | 8,30E-02 | 0,38 | 4,83E-03 | 0,37 |
| hsa-miR-28-3p | 3,56E-01 | 0,44 | 1,24E-06 | 0,32 |
| hsa-miR-151a-3p | 5,87E-01 | 0,44 | 1,29E-15 | 0,20 |
| hsa-miR-1468-5p | 7,11E-02 | 0,39 | 8,61E-17 | 0,14 |
| hsa-miR-532-5p | 5,89E-03 | 0,71 | 4,37E-21 | 0,87 |
| hsa-miR-17-3p | 2,59E-01 | 0,59 | 1,23E-16 | 0,82 |
| hsa-miR-30a-3p | 5,84E-01 | 0,58 | 6,06E-03 | 0,44 |

Während die signifikantesten AD-miRNAs aus dem Panel aus Beispiel 1 auch dys-reguliert zwischen MS und AD waren, waren MCI-Patienten ähnlicher zu AD-Patienten.

Da dasselbe Panel an miRNAs auch zwischen MCI, MS und AD unterscheiden kann, kann gefolgert werden, dass die miRNAs in der gefundenen Signatur spezifisch für Alzheimer sind und entsprechend für eine frühe Detektion bzw. Diagnose der Erkrankung verwendet werden können.

## Patentansprüche

1. Verfahren zur Diagnose von Alzheimer in einem Patienten, umfassend:
Erstellen eines Expressionsprofils von miRNAs aus einer bereitgestellten Blutprobe eines Patienten, und
Vergleich des Expressionsniveaus einer miRNA, die eine Sequenz umfasst, die ausgewählt ist aus hsa-miR-345-5p, und/oder die eine Sequenz umfasst, die von dieser Sequenz um jeweils bis zu 3 Basen, bevorzugt bis zu 2 Basen, weiter bevorzugt 1 Base abweicht, mit einem Referenzniveau, wobei der Vergleich die Diagnose einer Erkrankung mit Alzheimer ermöglicht.

2. Verfahren nach Anspruch 1, wobei zudem das Expressionsniveau mindestens einer miRNA, die eine Sequenz umfasst, die ausgewählt ist aus der Gruppe, bestehend aus hsa-miR-5006-3p, hsa-miR-7848-3p, hsa-miR-6817-3p, hsa-miR-361-5p, hsa-miR-3157-3p, hsa-miR-4482-3p, hsa-miR-28-3p, hsa-miR-151a-3p, hsa-miR-1468-5p, hsa-miR-532-5p, hsa-miR-17-3p, und hsa-miR-30a-3p, und/oder die eine Sequenz umfasst, die von diesen Sequenzen um jeweils bis zu 3 Basen, bevorzugt bis zu 2 Basen, weiter bevorzugt 1 Base abweicht, mit einem Referenzniveau verglichen wird, wobei der Vergleich die Diagnose einer Erkrankung mit Alzheimer ermöglicht.

3. Verfahren nach einem der vorigen Ansprüche, wobei der Patient ein Mensch ist.

4. Verfahren nach einem der vorigen Ansprüche, wobei in mindestens 2, 3, 4, 5, 6, 7, 8, 9, 10, 11, 12 oder 13 miRNAs, die eine Sequenz umfassen, die ausgewählt ist aus der Gruppe, bestehend aus hsa-miR-345-5p, hsa-miR-5006-3p, hsa-miR-7848-3p, hsa-miR-6817-3p, hsa-miR-361-5p, hsa-miR-3157-3p, hsa-miR-4482-3p, hsa-miR-28-3p, hsa-miR-151a-3p, hsa-miR-1468-5p, hsa-miR-532-5p, hsa-miR-17-3p, und hsa-miR-30a-3p, und/oder die eine Sequenz umfassen, die von diesen Sequenzen um jeweils bis zu 3 Basen, bevorzugt bis zu 2 Basen, weiter bevorzugt 1 Base abweicht, eine Änderung im Expressionsniveau im Vergleich zu einem Referenzniveau beobachtet wird, wobei in mindestens einer miRNA, die eine Sequenz umfasst, die ausgewählt ist aus hsa-miR-345-5p, und/oder die eine Sequenz umfasst, die von dieser Sequenz um jeweils bis zu 3 Basen, bevorzugt bis zu 2 Basen, weiter bevorzugt 1 Base abweicht, eine Änderung im Expressionsniveau im Vergleich zu einem Referenzniveau beobachtet wird.

5. Verfahren nach einem der vorigen Ansprüche, wobei die Erstellung des Expressionsprofils eine Nukleinsäurehybridisierung, eine Nukleinsäureamplifikation, eine Polymeraseextension, eine Sequenzierung, eine Massenspektrometrie, oder jegliche Kombination davon umfasst.

6. Verwendung einer miRNA, die eine Sequenz umfasst, die ausgewählt ist aus hsa-miR-345-5p, und/oder die eine Sequenz umfasst, die von dieser Sequenz um jeweils bis zu 3 Basen, bevorzugt bis zu 2 Basen, weiter bevorzugt 1 Base abweicht, als Marker für die Diagnose von Alzheimer.

## Claims

1. Method for diagnosing Alzheimer's disease in a patient, comprising:
creating an expression profile of miRNAs from a blood sample provided from a patient, and
comparing the expression level of an miRNA which comprises a sequence selected from hsa-miR-345-5p and/or
which comprises a sequence deviating from this sequence by, in each case, up to 3 bases, preferably up to 2 bases, further preferably 1 base, with a reference level,
the comparison allowing the diagnosis of Alzheimer's disease.

2. Method according to Claim 1, wherein the expression level of at least one miRNA which comprises a sequence selected from the group consisting of hsa-miR-5006-3p, hsa-miR-7848-3p, hsa-miR-6817-3p, hsa-miR-361-5p, hsa-miR-3157-3p, hsa-miR-4482-3p, hsa-miR-28-3p, hsa-miR-151a-3p, hsa-miR-1468-5p, hsa-miR-532-5p, hsa-miR-17-3p and hsa-miR-30a-3p and/or which comprises a sequence deviating from this sequence by, in each case, up to 3 bases, preferably up to 2 bases, further preferably 1 base, is additionally compared with a reference level, the comparison allowing the diagnosis of Alzheimer's disease.

3. Method according to either of the preceding claims, wherein the patient is a human.

4. Method according to any of the preceding claims, wherein a change in the expression level in comparison with a reference level is observed in at least 2, 3, 4, 5, 6, 7, 8, 9, 10, 11, 12 or 13 miRNAs which comprise a sequence selected from the group consisting of hsa-miR-345-5p, hsa-miR-5006-3p, hsa-miR-7848-3p, hsa-miR-6817-3p, hsa-miR-361-5p, hsa-miR-3157-3p, hsa-miR-4482-3p, hsa-miR-28-3p, hsa-miR-151a-3p, hsa-miR-1468-5p, hsa-miR-532-5p, hsa-miR-17-3p and hsa-miR-30a-3p and/or which comprise a sequence deviating from these sequences by, in each case, up to 3 bases, preferably up to 2 bases, further preferably 1 base, a change in the expression level in comparison with a reference level being observed in at least one miRNA which comprises a sequence selected from hsa-miR-345-5p and/or which comprises a sequence deviating from this sequence by, in each case, up to 3 bases, preferably up to 2 bases, further preferably 1 base.

5. Method according to any of the preceding claims, wherein creating the expression profile comprises nucleic acid hybridization, nucleic acid amplification, polymerase extension, sequencing, mass spectrometry, or any combination thereof.

6. Use of an miRNA which comprises a sequence selected from hsa-miR-345-5p and/or which comprises a sequence deviating from this sequence by, in each case, up to 3 bases, preferably up to 2 bases, further preferably 1 base, as marker for the diagnosis of Alzheimer's disease.

## Revendications

1. Procédé pour le diagnostic de la maladie d'Alzheimer chez un patient, comprenant : l'établissement d'un profil d'expression de miARN provenant d'un échantillon sanguin d'un patient mis à disposition et comparaison du niveau d'expression d'un miARN, qui comprend une séquence choisie parmi hsa-miR-345-5p et/ou qui comprend une séquence qui s'écarte de cette séquence à chaque fois de jusqu'à 3 bases, de préférence de jusqu'à 2 bases, plus préférablement de 1 base, à un niveau de référence, la comparaison permettant le diagnostic de la maladie d'Alzheimer.

2. Procédé selon la revendication 1, le niveau d'expression d'au moins un miARN, qui comprend une séquence, qui est choisie dans le groupe constitué par hsa-miR-5006-3p, hsa-miR-7848-3p, hsa-miR-6817-3p, hsa-miR-361-5p, hsa-miR-3157-3p, hsa-miR-4482-3p, hsa-miR-28-3p, hsa-miR-151a-3p, hsa-miR-1468-5p, hsa-miR-532-5p, hsa-miR-17-3p et hsa-miR-30a-3p et/ou qui comprend une séquence qui s'écarte de ces séquences à chaque fois de jusqu'à 3 bases, de préférence de jusqu'à 2 bases, plus préférablement de 1 base, étant en outre comparé à un niveau de référence, la comparaison permettant le diagnostic de la maladie d'Alzheimer.

3. Procédé selon l'une quelconque des revendications précédentes, le patient étant un être humain.

4. Procédé selon l'une quelconque des revendications précédentes, une modification du niveau d'expression par rapport à un niveau de référence étant observée dans au moins 2, 3, 4, 5, 6, 7, 8, 9, 10, 11, 12 ou 13 miARN, qui comprennent une séquence qui est choisie dans le groupe constitué par hsa-miR-345-5p, hsa-miR-5006-3p, hsa-miR-7848-3p, hsa-miR-6817-3p, hsa-miR-361-5p, hsa-miR-3157-3p, hsa-miR-4482-3p, hsa-miR-28-3p, hsa-miR-151a-3p, hsa-miR-1468-5p, hsa-miR-532-5p, hsa-miR-17-3p et hsa-miR-30a-3p et/ou qui comprennent une séquence qui s'écarte de ces séquences à chaque fois de jusqu'à 3 bases, de préférence de jusqu'à 2 bases, plus préférablement de 1 base, une modification du niveau d'expression par rapport à un niveau de référence étant observée dans au moins un miARN, qui comprend une séquence qui est choisie parmi hsa-miR-345-5p et/ou qui comprend une séquence qui s'écarte de cette séquence à chaque fois de jusqu'à 3 bases, de préférence de jusqu'à 2 bases, plus préférablement de 1 base.

5. Procédé selon l'une quelconque des revendications précédentes, l'établissement du profil d'expression comprenant une hybridation d'acide nucléique, une amplification d'acide nucléique, une extension par polymérase, un séquençage, une spectrométrie de masse ou une combinaison quelconque de ceux-ci.

6. Utilisation d'un miARN qui comprend une séquence choisie parmi hsa-miR-345-5p et/ou qui comprend une séquence qui s'écarte de cette séquence à chaque fois de jusqu'à 3 bases, de préférence de jusqu'à 2 bases, plus préférablement de 1 base, comme marqueur pour le diagnostic de la maladie d'Alzheimer.
